# EUROPEAN PATENT SPECIFICATION

(11) **EP 3 955 930 B1**
(45) Date of publication and mention of the grant of the patent: **06.11.2024**
(21) Application number: 20791696.6
(22) Date of filing: 14.04.2020
(51) Int. Cl.: A61K 31/546, C07K 14/22

(54) **METHODS FOR PREDICTING NEISSERIA SPP. SUSCEPTIBILITY TO CEFIXIME**
VERFAHREN ZUR VORHERSAGE DER EMPFINDLICHKEIT VON NEISSERIA SPP. GEGENÜBER CEFIXIMS
PROCÉDÉS DE PRÉDICTION DE SUSCEPTIBILITÉ DE NEISSERIA SPP. À CEFIXIME

(30) Priority: 18.04.2019 US 201962835746 P
(43) Date of publication of application: 23.02.2022
(73) Proprietor: The Regents of University of California, Oakland, CA 94607-5200 (US)
(72) Inventor: KLAUSNER, Jeffrey D., Los Angeles, California 90024-4201 (US)
(74) Representative: Bartle Read
(86) International application number: PCT/US2020/028064
(87) International publication number: WO 2020/214557

(56) References cited:
- WO-A1-2016/066306
- US-A1- 2017 260 570
- US-A1- 2018 355 410
- R. LINDBERG ET AL: "Neisseria gonorrhoeae Isolates with Reduced Susceptibility to Cefixime and Ceftriaxone: Association with Genetic Polymorphisms in penA, mtrR, porB1b, and ponA", ANTIMICROBIAL AGENTS AND CHEMOTHERAPY, vol. 51, no. 6, 9 April 2007 (2007-04-09), US, pages 2117 - 2122, XP055492887, ISSN: 0066-4804, DOI: 10.1128/AAC.01604-06
- HARRISON ODILE B ET AL: "Genomic analyses ofNeisseria gonorrhoeaereveal an association of the gonococcal genetic island with antimicrobial resistance", JOURNAL OF INFECTION, ACADEMIC PRESS, LONDON, GB, vol. 73, no. 6, 26 August 2016 (2016-08-26), pages 578 - 587, XP029821435, ISSN: 0163-4453, DOI: 10.1016/J.JINF.2016.08.010
- ALLEN VANESSA G. ET AL: "Neisseria gonorrhoeae Treatment Failure and Susceptibility to Cefixime in Toronto, Canada", JAMA THE JOURNAL OF THE AMERICAN MEDICAL ASSOCIATION, vol. 309, no. 2, 9 January 2013 (2013-01-09), US, pages 163, XP093031791, ISSN: 0098-7484, DOI: 10.1001/jama.2012.176575
- TAKAHATA SHO ET AL: "Amino Acid Substitutions in Mosaic Penicillin-Binding Protein 2 Associated with Reduced Susceptibility to Cefixime in Clinical Isolates of Neisseria gonorrhoeae", ANTIMICROBIAL AGENTS AND CHEMOTHERAPY, vol. 50, no. 11, 1 November 2006 (2006-11-01), US, pages 3638 - 3645, XP093031820, ISSN: 0066-4804, Retrieved from the Internet <URL:https://www.ncbi.nlm.nih.gov/pmc/articles/PMC1635225/pdf/0626-06.pdf> DOI: 10.1128/AAC.00626-06
- NABU SUNANTA ET AL: "Proteochemometric model for predicting the inhibition of penicillin-binding proteins", JOURNAL OF COMPUTER-AIDED MOLECULAR DESIGN, SPRINGER NETHERLANDS, NL, vol. 29, no. 2, 26 October 2014 (2014-10-26), pages 127 - 141, XP035429813, ISSN: 0920-654X, [retrieved on 20141026], DOI: 10.1007/S10822-014-9809-0
- KUBANOVA A A ET AL: "The role of some individual amino acid substitutions in penicillin-binding protein (PBP2) ofNeisseria gonorrhoeaein the emergence of resistance to ceftriaxone", MOLECULAR BIOLOGY : COVER-TO-COVER TRANSLATION = MOLEKULYARNAYA BIOLOGIYA, ACADEMY OF SCIENCES OF THE USSR, RU, vol. 48, no. 6, 17 December 2014 (2014-12-17), pages 858 - 863, XP035462913, ISSN: 0026-8933, [retrieved on 20141217], DOI: 10.1134/S0026893314060119
- NABU ET AL.: "Proteochemometric Model for Predicting the Inhibition of Penicillin-Binding Proteins", JOURNAL OF COMPUTER-AIDED MOLECULAR DESIGN, vol. 29, no. 2, 26 October 2014 (2014-10-26), pages 127 - 141, XP035429813, DOI: 10.1007/s10822-014-9809-0
- YAHARA ET AL.: "Genomic surveillance of Neisseria gonorrhoeae to investigate the distribution and evolution of antimicrobial-resistance determinants and lineages", MICROBIAL GENOMICS, vol. 4, no. 8, 31 July 2018 (2018-07-31), pages 1 - 13, XP055750314, DOI: 10.1099/mgen.0.000205

## Description

### CROSS-REFERENCE TO RELATED APPLICATIONS

This application claims the benefit of U.S. Patent Application No. 62/835,746, filed April 18, 2019.

### REFERENCE TO A SEQUENCE LISTING SUBMITTED VIA EFS-WEB

The content of the ASCII text file of the sequence listing named "20200414_034044_202W01_ST25" which is 5.18 kb in size was created on April 6, 2020 and electronically submitted via EFS-Web.

### ACKNOWLEDGEMENT OF GOVERNMENT SUPPORT

This invention was made with Government support under Grant Number AI117256, awarded by the National Institutes of Health. The Government has certain rights in the invention.

### BACKGROUND OF THE INVENTION

### 1. FIELD OF THE INVENTION

The field generally relates to predicting whether a *Neisseria* species is susceptible to cefixime compounds and treating infections by *Neisseria* species with cefixime compounds.

### 2. DESCRIPTION OF THE RELATED ART

*Neisseria (N.) gonorrhoeae* has become resistant to most if not all available antibiotics. Cefixime, a third-generation oral cephalosporin, is a highly effective and safe single dose treatment for susceptible cases of gonorrhea. However, in the last two decades, gonococcal strains with decreased susceptibility to cefixime and cases of clinical treatment failure with cefixime have been reported worldwide.

As the organism has developed resistance to multiple classes of antibiotics such as sulfas, penicillins, tetracyclines, fluoroquinolones, and macrolides, the third-generation extended-spectrum cephalosporins, like cefixime, are among the few reliable efficacious treatment options left. Cefixime is a highly useful antibiotic used for the treatment of gonorrhea. *N. gonorrhoeae* remains susceptible to cefixime in most but not all countries. Currently, the World Health Organization (WHO) recommends cefixime, in combination with azithromycin, as dual therapy for oropharyngeal, genital, and anorectal gonococcal infection. In settings where local resistance data confirm cefixime susceptibility, the WHO recommends cefixime in a single dose for genital and anorectal gonococcal infection. In the United States, the Centers for Disease Control and Prevention also recommends cefixime, in combination with azithromycin, as an alternative regimen where ceftriaxone is not available. In the United Kingdom, oral cefixime in combination with azithromycin is also recommended in penicillin-allergic patients for whom intramuscular injection is contraindicated or refused. Cefixime has a serum half-life of 3 to 4 hours in patients with normal renal function, high bioavailability after a single oral dose and is very well tolerated even in penicillin allergic patients. However, in the last two decades, various investigators have reported cases of *N. gonorrhoeae* infection with strains that have decreased susceptibility to cefixime. Furthermore, in the past 10 years, cases of *N. gonorrhoeae* treatment failure in patients treated with cefixime have also been reported in Japan, Norway, UK, South Africa, France, Australia, and Canada. Various research teams and governmental institutions have expressed the need for more research on the mechanisms of cefixime resistance and the development of new tools to predict cefixime susceptibility.

### SUMMARY OF THE INVENTION

In some embodiments, the present invention provides an in vitro method for inhibiting the growth of a given *Neisseria* species, which comprises
(A) determining the presence or absence of amino acid alterations at amino acid positions 345, 375-377, 501, 542, and 551 in the penicillin-binding protein 2 (PBP2) of the given *Neisseria* species;
(B) characterizing the given *Neisseria* species as being susceptible to cefixime compounds where
   amino acid alterations at amino acid positions 345, 375-377, 501, 542, and 551 are absent; and
(C) contacting the given *Neisseria* species with a cefixime compound where the given *Neisseria* species is characterized as being susceptible to cefixime compounds, and contacting the given *Neisseria* species with an antibacterial other than cefixime compounds where the given *Neisseria* species is characterized as being resistant to cefixime compounds.

In some embodiments, the present invention provides an in vitro method for characterizing whether a given *Neisseria* species is susceptible to a cefixime compound, which comprises
(A) determining the presence or absence of amino acid alterations at amino acid positions 345, 375-377, 501, 542, and 551 of its penicillin-binding protein 2 (PBP2), and
(B) characterizing the given *Neisseria* species as being susceptible to the cefixime compound where amino acid alterations at amino acid positions 345, 375-377, 501, 542, and 551 are absent.

In some embodiments, the given *Neisseria* species may be a *Neisseria* gonorrhoeae strain.

In some embodiments, the cefixime compound may be cefixime.

In some embodiments, the antibacterial other than cefixime compounds may be selected from the following groups of antibiotics: Sulfonamides, Tetracyclines, Aminoglycosides, Macrolides, Ketolides, Quinolones, Lincomycins, and Glycopeptides.

Both the foregoing general description and the following detailed description are exemplary and explanatory only and are intended to provide further explanation of the invention as claimed. The accompanying drawings are included to provide a further understanding of the invention and are incorporated in and constitute part of this specification, illustrate several embodiments of the invention, and together with the description explain the principles of the invention.

### DESCRIPTION OF THE DRAWINGS

This invention is further understood by reference to the drawings wherein:

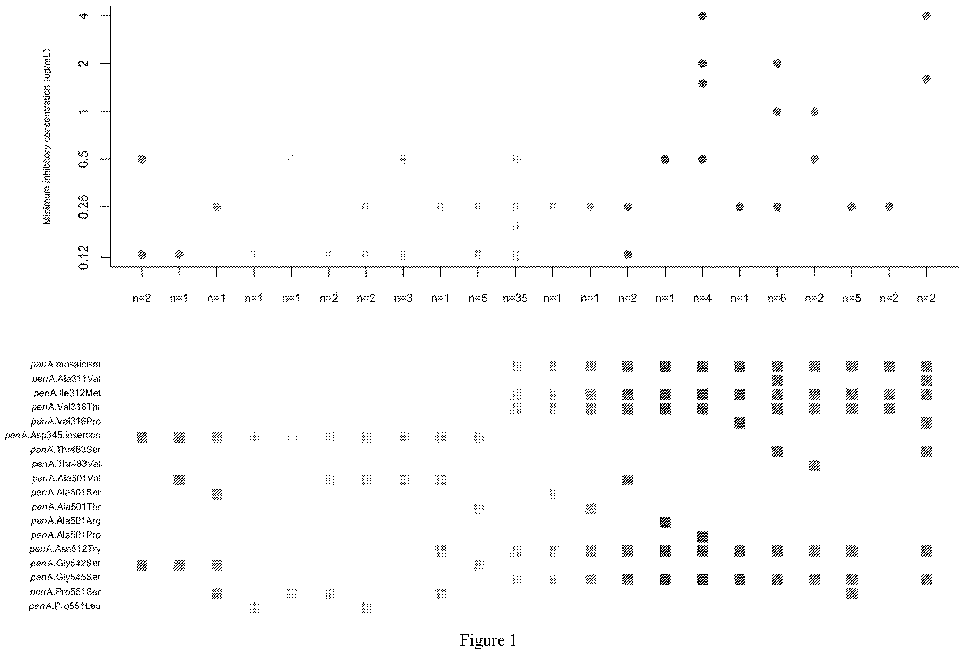

Figure 1 graphically summarizes cefixime MIC of *N. gonorrhoeae* strains by different combinations of cefixime decreased susceptibility-related penA amino acid alterations (amino acid alterations encoded by the *penA* gene of the given strain). Minimum inhibitory concentrations are on the y-axis. Combinations of *penA* amino acid alterations associated with cefixime decreased susceptibility are on the x-axis; different colors indicate different mutation combinations.

### DETAILED DESCRIPTION OF THE INVENTION

The penicillin-binding protein 2 (PBP2) encoded by *penA* gene is the primary target of cefixime antimicrobial activity. *Neisseria gonorrhoeae* strains having a cefixime minimum inhibitory concentration (MIC) ≥ 0.12 µg/mL are significantly more likely to fail treatment with cefixime than strains with an MIC < 0.12 µg/ml. Therefore, as provided herein, *Neisseria gonorrhoeae* strains having a cefixime MIC ≥ 0.12 µg/mL are characterized as having a decreased susceptibility (i.e., resistance) to cefixime and strains having a cefixime MIC < 0.12 µg/mL are characterized as being susceptible to cefixime.

Disclosed herein are genetic profiles of *Neisseria gonorrhoeae* strains that exhibit resistance to cefixime as determined from a literature review and analysis of PBP2 and *penA* contributing to cefixime resistance in *N. gonorrhoeae.* As disclosed herein, either of the following patterns of alterations lead to cefixime resistance: 1) Mosaic *penA* types (e.g., alterations in the amino acid region 375-377 of wildtype PBP2 (Accession No. M32091.1)) having characteristic polymorphisms I312M, V316T, N512Y, G545S, and 2) Non-mosaic *penA* types having any combination of amino acid alterations at amino acid positions A501, G542, and P551 of wildtype PBP2.

### Susceptibility Assays

In some embodiments, the present invention is directed to in vitro methods for characterizing whether a given *Neisseria* species, such as a given *Neisseria gonorrhoeae* strain, is susceptible to a cefixime compound, e.g., cefixime, which comprises (A) determining the presence or absence of one or more amino acid alterations at amino acid positions 345, 375-377, 501, 542, and 551 of its PBP2, and (B1) characterizing the given *Neisseria* species as being resistant to the cefixime compound where one or more alterations at amino acid positions 345, 375-377, 501, 542, and 551 are present, or (B2) characterizing the given *Neisseria* species as being susceptible to the cefixime compound where amino acid alterations at amino acid positions 345, 375-377, 501, 542, and 551 are absent.

In some embodiments, the present invention is directed to in vitro methods for characterizing whether a given *Neisseria* species, such as a given *Neisseria gonorrhoeae* strain, is susceptible to cefixime, which comprises (A) determining the presence or absence of one or more amino acid alterations at amino acid positions 345, 375-377, 501, 542, and 551 of its PBP2, and (B 1) characterizing the given *Neisseria* species as being resistant to cefixime where one or more alterations at amino acid positions 345, 375-377, 501, 542, and 551 are present, or (B2) characterizing the given *Neisseria* species as being susceptible to cefixime where amino acid alterations at amino acid positions 345, 375-377, 501, 542, and 551 are absent.

In some embodiments, the present invention is directed to in vitro methods for characterizing whether a given *Neisseria* species, such as a given *Neisseria gonorrhoeae* strain, is susceptible to cefixime, which comprises (A) determining the presence or absence of one or more amino acid alterations at amino acid positions 345, 375-377, 501, 542, and 551 of its PBP2, and (B 1) characterizing the given *Neisseria* species as being resistant to cefixime where one or more amino acid alterations at amino acid positions 501, 542, and 551 are present, (B2) characterizing the given *Neisseria* species as being resistant to cefixime where one or more amino acid alterations at amino acid positions 375-377 and one or more amino acid alterations at amino acid positions 312, 316, 512, and 545 are present, or (B3) characterizing the given *Neisseria* species as being susceptible to cefixime where one or more amino acid alterations at amino acid positions 501, 542, and 551 are absent, and a combination of (a) one or more amino acid alterations at amino acid positions 375-377 and (b) one or more amino acid alterations at amino acid positions 312, 316, 512, and 545 is also absent.

In some embodiments, at least one of the amino acid alterations is an amino acid substitution. In some embodiments, the amino acid substitution is A311V, A376X, A501P, A501R, A501S, A501T, A501V, E377X, G375X, G542S, G545S, I312M, N512Y, P551A, P551G, P551L, P551S, T483S, V316P, or V316T, wherein each X are independently any amino acid residue. In some embodiments, the amino acid insertion is an insertion after or before the residue at amino acid position 345. In some embodiments, the amino acid insertion is Asp inserted after or before amino acid position 345 (herein denoted as "345insD") based on the wildtype PBP2 sequence. In some embodiments, the one or more amino acid alterations is 345insD; N512Y; 345insD and A501T; 345insD and A501V; 345insD and G542S; 345insD and P551A; 345insD and P551L; 345insD and P551S; I312M and V316T; 345insD, A501S, and G542S; 345insD, A501T, and G542S; 345insD, A501T, and P551L; 345insD, A501V, and G542S; 345insD, A501V, and P551A; 345insD, A501V, and P551G; 345insD, A501V, and P551L; 345insD, A501V, and P551S; N512Y, G545S, and P551A; T483S, N512Y, and G545S; 345insD, A501S, G542S, and P551S; 345insD, A501V, N512Y, and P551S; I312M, V316P, N512Y, and G545S; I312M, V316T, N512Y, and G545S; V316T, N512Y, G545S, and P551A; A311V, I312M, V316P, N512Y, and G545S; A311V, I312M, V316T, N512Y, and G545S; I312M, V316T, A501P, N512Y, and G545S; I312M, V316T, A501R, N512Y, and G545S; I312M, V316T, A501S, N512Y, and G545S; I312M, V316T, A501T, N512Y, and G545S; I312M, V316T, A501V, N512Y, and G545S; I312M, V316T, N512Y, G545S, and P551S; I312M, V316T, T483V, N512Y, and G545S; A311V, I312M, V316P, T483S, N512Y, and G545S; or A311V, I312M, V316T, T483S, N512Y, and G545S. In some embodiments, the one or more amino acid alterations comprise at least one of G375X, A376X, and E377X, wherein each X are independently any amino acid, and at least one of A311V, A501P, A501R, A501S, A501T, A501V, G542S, G545S, I312M, N512Y, P551A, P551G, P551L, P551S, T483S, V316P, V316T, and 345insD.

As provided herein, amino acid alterations at amino acid positions 345, 375-377, 501, 542, and 551 of the PBP2 of a given *Neisseria* species are based on an optimal alignment of the PBP2 sequence of the given *Neisseria* species with the wildtype PBP2 sequence (Accession No. AAA25463.1 (SEQ ID NO: 1) (which is encoded by Accession No. M32091.1)) and using the amino acid numbering of the wildtype PBP2 sequence. Optimal alignment of sequences for comparison can be conducted, e.g., by the local homology algorithm of Smith & Waterman, Adv Appl Math 2:482 (1981), by the homology alignment algorithm of Needleman & Wunsch, J Mol Biol 48:443 (1970), by the search for similarity method of Pearson & Lipman, PNAS USA 85:2444 (1988), by computerized implementations of these algorithms (GAP, BESTFIT, FASTA, and TFASTA in the Wisconsin Genetics Software Package, Genetics Computer Group, 575 Science Dr., Madison, WI), or by visual inspection.

In some embodiments, the presence or absence of any one or more of the above amino acid alterations at amino acid positions 345, 375-377, 501, 542, and 551 of the PBP2 protein of the given *Neisseria* species (as compared to the wildtype PBP2) are determined by assaying the *penA* gene of the given *Neisseria* species for mutations that result in the amino acid alterations.

The sensitivity of assay methods for characterizing whether a given *Neisseria gonorrhoeae* strain is susceptible to cefixime based on the absence of an amino acid alteration in amino acid positions 375-377 combined with an amino acid alteration at amino acid positions 345, 501, 542, and 551 of its PBP2 as compared to the wildtype sequence is predicted to be at least about 99.5%.

### EXAMPLES

### Literature Review and Analysis

Articles published on PubMed from January 01, 1995 to November 01, 2018 were searched using the search terms, *"Neisseria gonorrhoeae",* "cefixime", and "molecular" and the relevant hits were reviewed. A total of 74 articles from that search were identified. Articles that presented epidemiological or experimental evidence of certain molecular alterations contributing to cefixime decreased susceptibility in *N. gonorrhoeae* totaled 25 reports. All *N. gonorrhoeae* strains with reported MIC of ≥ 0.12 µg/mL and specific *penA* alterations are included in Table 1 along with their specific MIC plus the time and location of collection.

**Table 1. Summary of penA alterations in reported gonococcal strains with cefixime MIC ≥ 0.12 µg/mL**

| **Reference** | **Country of collection** | **Year** | **Frequency** | **CFX MIC (µg/mL)** | ***pen*A** t**ype** | **Amino Acid Alterations**¹ | | | | | | | | | | |
|---|---|---|---|---|---|---|---|---|---|---|---|---|---|---|---|---|
| | | | | | | **mosaicism** | **A311** | **I312** | **V316** | **D345ins** | **T483** | **A501** | **N512** | **G542** | **G545** | **P551** |
| **14** | Japan | 2001 | 8/55² 8/55² | 0.125 | 1~9³ | No | | | | Yes | | V⁴ | | S⁵ | | |
| **15** | Argentina | 2009-13 | 1/1987 | 0.125 | 5 | No | | | | Yes | | | | S | | |
| **8** | Vietnam | 2011 | 1/108 | 0.125 | 5+⁶ | No | | | | Yes | | V | | S | | |
| **16** | Canada | 2001 -10 | 1/155 | 0.5 | 5+ | No | | | | Yes | | | | S | | |
| **9** | ^{:}(WHO reference strain L) | | | 0.25 | 7 | No | | | | Yes | | S | | S | | S |
| **15** | Argentina | 2009-13 | 1/1987 | 0.125 | 9 | No | | | | Yes | | | | | | |
| | | | 1/1987 | 0.5 | 12 | No | | | | Yes | | | | | | S |
| **16** | Canada | 2001-10 | 3/155 | ≥ 0.125 | 12+ | No | | | | Yes | | | | | | S |
| **17** | Spain | 2013 | 13/329 | ≥ 0.125 | 12+⁷ | No | | | | Yes | | T | | | | L |
| | | | 2/329 | | | | | | | Yes | | | | S S | | |
| | | | 2/329 | | | | | | | Yes | | | | | | |
| | | | 1/329 | | | | | | | Yes | | | | | | |
| | | | 1/329 | | | | | | | Yes | | S | | S | | |
| **18** | China | 2014-15 2014-15 | 3/126 | 0.125 | 13 | No | | | | Yes | | V V | | | | S |
| **15** | Argentina | 2009-13 | 1/1987 | 0.125 1 | 13 | No | | | | Yes | | | | | | S |
| **10** | Korea | 2011-2013 | 16/210 | 0.12 | 13 | No | | | | Yes | | V | | | | S |
| | | | 13/210 | 0.25 | | | | | | Yes | | V | | | | |
| | | | 2/210 | 0 .5 | | | | | | Yes | | V | | | | S |
| **18** | China | 2014-15 | 1/126 | 0.25 | 13+ | No | | | | Yes | | V | Y | | | S |
| **16** | Canada | 2001-10 | 1/155 | 0.125 | 13+ | No | | | | Yes | | V | | | | S |
| **8** | Vietnam | 2011 | 1/108 | 0.25 | 18 | No | | | | Yes | | T | | S | | |
| | | | 2/108 | 0.125 | | | | | | Yes | | T | | S | | |
| | | | 1/108 | 0.125 | | | | | | Yes | | T | | S | | |
| | | | 2/108 | 0.125 | | | | | | Yes | | | | | | |
| | | | 1/108 | 0.125 | | | | | | Yes | | | | S | | |
| **18** | China | 2014-15 | 1/126 | 0.125 | 21 | No | | | | Yes | | V | | | | |
| **19** | Japan | 2002 | 20/58 | 0.25 | 10 | Yes | | M | T | | | | Y | | S | |
| | | | 4/58 | | | | | M | T | | | | Y | | S | |
| **14** | Japan | 2001 | 37/55⁸ | ≥ 0.5 | 10 | Yes | | M | T | | | | Y | | S | |
| | | | 8/55⁹ | 0.25 | | | | M | T | | | | | | S | |
| | | | 2/55¹⁰ | 0.125 | | | | M | T | | | | Y | | S | |
| **18** | China | 2014-15 | 3/126 | 0.25 | 10¹¹ | Yes | | M | T | | | | Y | | S | |
| **15** | Argentina | 2009-13 | 3/1987 | 0.5 | 10 | Yes | | M | T | | | | Y | | S | |
| | Korea | 2011-2013 | 3/210 | 0.25 | 10 | Yes | | M M | T | | | | Y | | S | |
| | | | 1/210 | 0.5 | | | | | T | | | | Y | | S | |
| **70** | (Modified laboratory stain) | | | > 0.25 | 10 | Yes | | M | T | | | | Y | | S | |
| **9** | (WHO reference strain K) | | | 0.5 | 10 | Yes | | M | T | | | | Y | | S | |
| | (Modified laboratory strain) | | | 0 .5 | 10¹² | Yes | | M | T | | | | Y | | S | |
| **16** | Canada | 2001-10 | 12/155 | ≥ 0.25 | 10 | Yes | | M | T | | | | Y | | S | |
| **20** | Canada | 2008 | 8/149 | 0.25 | 10¹³ | Yes | | M | T | | | | Y | | S | |
| | Japan | 1998-2007 | 25/36¹⁴ | 0.25 | 10 | Yes | | | | | | | Y | | | |
| | | 2003-04 | 5/36¹⁵ | 0.5 | | | | M M | T | | | | | | | |
| **19** | Japan | 2002 | 4/58 | 0.12 | 10 | Yes | | M | T | | | | Y | | S | |
| | (Modified laboratory stains) | | | 0.25 | | Yes | | M | T | | | S | Y | | S | |
| **51** | | | | 0.25 | 10+¹⁶ | | | M | T | | | T | Y | | S | |
| | | | | 0.25 | | | | M | T | | | | Y | | S | |
| | | | | 0.5 | | | | M | T | | | R | Y | | S | |
| | | | | 0.5 | | | | M | | | | P | Y | | S | |
| **50** | (Modified laboratory | | | 0.125 | 10+¹⁷ | Yes | | M | T | | | | Y | | S | |
| | | | | > 0.125 | | | V | M | T | | | | Y | | S | |
| | | | | 0.25 | | | | M | P | | | | Y | | S | |
| | | | | 0 .25 | | | V | M | T | | S | | Y | | S | |
| | | | | > 0.5 | | | V | M | P | | | | Y | | S | |
| | | | | > 1.5 | | | V | M | P | | S | | Y | | S | |
| **11** | Japan | 2003 | 1/36¹⁸ | 0.5 | 30 | Yes | | M | T | | | | Y | | S | |
| | | 2003 | 1/36¹⁹ | 1 | | | | M | T | | V | | Y | | S | |
| | | 2001 | 1/36²⁰ | 0.25 | 31 | Yes | | M | T | | | | Y | | S | |
| | | 2005 | 1/36²¹ | 0.25 | 32 | Yes | | M | T | | | | Y | | S | |
| | | 2005 | 1/36²² | 0.5 | | | | M | | | | | Y | | S | |
| **15** | Argentina | 2009 2009-13 | 15/1987 | 0 .25 | 34 | Yes | | M | T | | | | Y | | S | |
| | | | 20/1987 | 0.125 | | | | M | T | | | | Y | | S | |
| **21** | Italy | 2011 -2014 | 45/50²³ | > 0.125 | 34 | Yes | | M | T | | | | Y | | S | |
| **17** | Spain | 2013 | 28/329 | ≥ 0.125 | 34 | Yes | | M | T | | | | Y | | S | |
| **12** | Japan | 2011 | Case report | 0.125 | 34 | Yes | | M | | | | | Y | | S | |
| **13** | Ireland | 2014 2014-16 | 10/608 | 0.125 | 34 | Yes | | | T | | | | Y | | S | |
| **22** | Slovenia | 2006-2012 | 28/194 | 0.125 | 34 | Yes | | | T | | | | Y | | S | |
| **23** | Switzerland | 2010 | 1/34 | 0.19 | 34 | Yes | | | T | | | | Y | | S | |
| | | 2011 | 1/34 | _{:}0.19 | | | | | | | | | Y | | S | |
| | | | 1/34 | 0.125 | | | | | T | | | | Y | | S | |
| | | | 1/34 | 0.125 | | | | M | T | | | | Y | | S | |
| **70** | (Modified laboratory strain) | | | > 0.125 | 34 | Yes | | M ! | T | | | | Y | | S | |
| **28** | South Africa | 2012 | Case report | 0.25 | 34 | Yes | | M | T | | | | Y | | S | |
| **24** | Canada | 2010-11 | 7/9²⁴ | 0.12 | 34 | Yes | | M | T | | | | Y | | S | |
| **20** | Canada | 2008 | 1/149 | 0.125 | 34 | Yes | | | T | | | | Y | | S | |
| | | | 1/149 | 0.25 | | | | M | T | | | | Y | | S | |
| **25** | USA | 2008 | Case report | 0.25 | 34 | Yes | | M | T | | | | Y | | S | |
| | | | | 0.125 | | | | M | T | | | | Y | | S | |
| **21** | Italy | 2011-2014 | 1/50²⁵ | > 0.125 | 34+ | Yes | | M | T | | | V | Y | | S | |
| **29** | Republic of Georgia | Did not report | Case report | 0.5 | 34+ | Yes | | M | T | | | | Y | | S | |
| **12** | Japan | 2012 | Case report | 0.125 0.125 | 34+ | Yes | | M | T | | | V | Y | | S | |
| **70** | (Modified laboratory strain) | | | > 0.35 | 34+ | Yes | | M | T | | | | Y | | S | S |
| | | | | > 0.5 | | | | M | T | | | S | Y | | S | |
| **26** | Spain | Did not | Case report | 1.5 | 34+ | Yes | | M | T | | | P | Y | | S | |
| **12** | Japan | 2011 | Case report | 0.25 0.25 | 34+ | Yes | | M | T | | | | Y | | S | S |
| | | | | 0.25 | | | | M | T | | | | Y | | S | S |
| | | | | 0.25 | | | | M | T | | | | Y | | S | S |
| | | 2012 | | 0.25 | | | | M | T | | | | Y | | S | S |
| | | | | 0.25 0.25 | | | | M | T | | | | Y | | S | S |
| **20** | Canada | 2008 | 2/149 | 0.25 | 35²⁶ | Yes | | M | T | | | | | | | |
| **30** | Japan | 2009 | Case report | 4 | 37 | Yes | V | M | P | | S | | Y | | S | |
| **50** | (Modified laboratory strain) | | | 1.6 | 37²⁷ | Yes | | M | P | | S | | Y | | S | |
| **30** | France | 2010 | Case report | 2 | 42 | Yes | | M | T | | | P | Y | | S | |
| **9** | France | 2010 | Case report | 4 | 42²⁸ | Yes | | M | T | | | P | Y | | S | |
| **30** | Japan | 2015 | Case | 1 | 60 | Yes | V | M | T | | S | | Y | | S | |
| | Japan | 2014 | report | 1 | | | V | M | T | | S | | Y | | | |
| | Denmark | 2017 | | 1 | | | V | M | T | | S | | Y | | S | |
| | Canada | 2017 | | | | | V | | T | | S | | Y | | S | |
| | Australia | 2013 | | 2 | 64 | Yes | V | M | T | | S | | Y | | S | |
| **21** | Italy | 2011-2014 | 4/50²⁹ | > 0.125 | not reported | Yes | (Amino acid sequence not reported) | | | | | | | | | |
| **16** | Canada | 2001-10 | 17/155 | ≥ 0.125 | not reported | Yes | | M | T | | | | Y | | S | |
| | | | 1/155 | 0.25 | | | | M | T | | | | | | | |
| **11** | Japan | 2003 | 1/36³⁰ | 0.5 | not reported | Yes | | M | T | | | | Y | | S | |

| | | | | | | | | | | | | | | | | |
|---|---|---|---|---|---|---|---|---|---|---|---|---|---|---|---|---|
| ¹ Single amino acid letters indicate the amino acid substitution of the corresponding amino acid relative to the wildtype PBP2 sequence when optimally aligned and using the amino acid numbering of the wildtype sequence. "Yes" for mosaicism indicates an amino acid sequence other than GAE at amino acid position 375-377 relative to the wildtype PBP2 sequence when optimally aligned and using the amino acid numbering of the wildtype sequence. ² Among isolates with cefixime MIC ≥ 0.125 µg/mL ³ In the manuscript, the authors reported the MIC values for a group of isolates with decreased susceptibility to cefixime with *pen*A type 1∼9. ⁴ A501V mutation present only in *pen*A 7&8; other *pen*A types are wild-type at the 501 position ⁵ G542S mutation present only in *penA* 4, 5, 7, 8 other *pen*A types are wild-type at the 542 position ⁶ In this entry and hereafter, "+" in the "*penA* type" column indicates that researchers reported the isolate having a *pen*A sequence closely resembling the reported type. ⁷ In the manuscript, the authors reported the *penA* type as closely resembling *penA* 36 ⁸ Among isolates with cefixime MIC ≥ 0.125 µg/mL ⁹ Among isolates with cefixime MIC ≥ 0.125 µg/mL ¹⁰ Among isolates with cefixime MIC ≥ 0.125 µg/mL ¹¹ In the manuscript, the authors reported the *pen*A type as *pen*A 35 ¹² In the manuscript, the authors reported the *penA* type as *pen*A 28 ¹³ In the manuscript, the authors reoorted the *pen*A type as *pen*A 35 ¹⁴ Among isolates with cefixime MIC ≥ 0.25 µg/mL ¹⁵ Among isolates with cefixime MIC ≥ 0.25 µg/mL ¹⁶ In the manuscript, the authors reported the *penA* type as closely resembling to *penA* 35 ¹⁷ In the manuscript, the authors reported the *penA* type as closely resembling to *penA* 35 ¹⁸ Among isolates with cefixime MIC ≥ 0.25 µg/mL ¹⁹ Among isolates with cefixime MIC ≥ 0.25 µg/mL ²⁰ Among isolates with cefixime MIC ≥ 0.25 µg/mL ²¹ Among isolates with cefixime MIC ≥ 0.25 µg/mL ²² Among isolates with cefixime MIC ≥ 0.25 µg/mL ²³ Among isolates with cefixime MIC ≥ 0.125 µg/mL ²⁴ Among isolates that failed clinical treatment ²⁵ Among isolates with cefixime MIC ≥ 0.125 µg/mL ²⁶ In the manuscript, the authors reported the *pen*A type as *pen*A 38 ²⁷ In the manuscript, the authors reported the *pen*A type as *pen*A 41 ²⁸ In the manuscript, the authors reported the *pen*A type as *pen*A 51 ²⁸ Among isolates with cefixime MIC ≥ 0.125 µg/mL ³⁰ Among isolates with cefixime MIC ≥ 0.25 µg/mL | | | | | | | | | | | | | | | | |

The nomenclature of *pen*A reflects the differences in amino-acid sequence rather than nucleotide sequence. Historically, each new amino-acid sequence gets a sequential whole number, and is classified into mosaic, semi-mosaic (alterations of either the first or second half of the *penA* gene only), non-mosaic (point mutations only), and wild-type (*pen*A peptide sequence identical to that of the *N. gonorrhoeae* reference strain M32091). Each different DNA sequence of an existing amino acid sequence gets a decimal number. For example, the 8th DNA sequence reported for *pen*A allele type 2 is assigned allele number *pen*A2.008. Based on an analysis of the literature in the art, there is conflicting nomenclature for the same *pen*A peptide sequence (see Table 2); likely due to the lack of a single, centralized database requiring new submissions of sequences to be compared with existing entries and subsequently given appropriate designations.

**Table 2. penA gene types with conflicting nomenclature**

| **Reference Strain** | ***pen*A type by NG-STAR** | ***pen*A type reported in literature** |
|---|---|---|
| 35/02 | 10 | mosaic-1 by Takahata *et al.* ¹⁹ |
| | | 28 by Unemo *et al.* ⁹ |
| | | 35 by Allen *et al.* ²⁶, Tomberg *et al.* ^{18,19}, Jiang *et al.* ^{18,20,50,51} |
| F98 | 42 | 51 by Unemo *et al.* ⁹ |
| | | 42 by Lahra *et al.* ³⁰ |
| H041 | 37 | 50 by Unemo *et al.* ⁹ |
| | | 41 by Tomberg *et al.* ¹⁸ |
| | | 37 by Lahra *et al.* ³⁰ |
| FA6140 | 12 | 36 by Allen *et al.²⁰,* Serra-Pladevall *et al.* ¹⁷ |
| / | 35 | 38 by Allen *et al.* ²⁶, Martin *et al.* ¹⁶ |

There was a general lack of consensus in the standard style of nucleotide/amino acid sequence reporting. Many sequences reported were truncated, leading to incomplete information that hindered data interpretation. To prevent misclassification, *penA* sequences from each research article were verified against the *penA* profiles from NG-STAR, a centralized, comprehensive, and publicly accessible database for standardized characterization of molecular alterations in *N. gonorrhoeae* worldwide. Multiple peptide sequence alignments of the *pen*A sequences using the Multiple Sequence Alignment tool by Clustal Omega (https://www.ebi.ac.uk/Tools/msa/clustalo/) were conducted.

Table 3 provides *the penA* profiles of *N. gonorrhoeae* strains that exhibit decreased susceptibility to cefixime:

**Table 3. penA profiles of Neisseria gonorrhoeae strains exhibiting resistance to cefixime**

| *PenA* type | Amino Acid Alteration at Amino Acid Position based on Wildtype PBP2¹ | | | | | | | | | | |
|---|---|---|---|---|---|---|---|---|---|---|---|
| | 375-377 | 311 | 312 | 316 | 345 | 483 | 501 | 512 | 542 | 545 | 551 |
| **Wildtype** | **Mosaicism** | **A** | **I** | **V** | **D** | **T** | **A** | **N** | **G** | **G** | **P** |
| 1 | No | | | | 345insD | | | | | | |
| 2 | No | | | | 345insD | | | | | | |
| 3 | No | | | | 345insD | | | | | | |
| 4 | No | | | | 345insD | | | | S | | |
| 5 | No | | | | 345insD | | | | S | | |
| 7 | No | | | | 345insD | | S | | S | | |
| 9 | No | | | | 345insD | | | | | | L |
| 10 | Yes | | M | T | | | | Y | | S | |
| 11 | No | | | | 345insD | | V | | | | L |
| 12 | No | | | | 345insD | | | | | | S |
| 13 | No | | | | 345insD | | V | | | | S |
| 14 | No | | | | 345insD | | | | | | |
| 15 | No | | | | | | | | | | |
| 17 | No | | | | 345insD | | V | | S | | |
| 18 | No | | | | 345insD | | T | | S | | |
| 19 | No | | | | 345insD | | | | | | |
| 21 | No | | | | 345insD | | V | | | | |
| 22 | No | | | | 345insD | | | | | | |
| 26 | Yes | | M | T | | | | Y | | S | |
| 27 | Yes | | M | T | | | | Y | | S | |
| 30 | Yes | | M | T | | V | | Y | | S | |
| 31 | Yes | | M | T | | | | Y | | S | |
| 32 | Yes | | M | T | | | | Y | | S | |
| 34 | Yes | | M | T | | | | Y | | S | |
| 35 | Yes | | M | T | | | | | | | |
| 37 | Yes | V | M | P | | S | | Y | | S | |
| 38 | Yes | | | | | | | | | | |
| 39 | Yes | | | | 345insD | | | | | | |
| 40 | No | | | | 345insD | | | | | | |
| 41 | No | | | | 345insD | | | | S | | |
| 42 | Yes | | M | T | | | P | Y | | S | |
| 43 | No | | | | 345insD | | V | | | | |
| 44 | No | | | | 345insD | | T | | | | L |
| 45 | No | | | | | | | | | | |
| 46 | No | | | | 345insD | | | | | | |
| 47 | Yes | | M | T | | | | | | | |
| 48 | No | | | | 345insD | | | | | | |
| 49 | No | | | | 345insD | | T | | | | |
| 50 | No | | | | 345insD | | | | | | A |
| 51 | Yes | | M | T | | | | Y | | S | |
| 52 | Yes | | M | T | | | | Y | | S | |
| 53 | Yes | | | T | | | | Y | | S | A |
| 54 | No | | | | 345insD | | V | | | | A |
| 55 | Yes | | M | T | | | | Y | | S | |
| 56 | No | | | | 345insD | | V | | | | G |
| 57 | No | | | | 345insD | | V | | | | A |
| 58 | Yes | | | | | | | Y | | S | A |
| 59 | Yes | | | | | S | | Y | | S | |
| 60 | Yes | V | M | T | | S | | Y | | S | |
| 61 | No | | | | 345insD | | | | | | L |
| 62 | Yes | | | | | | | Y | | | |
| 63 | Yes | | M | T | | | | | | | |
| 64 | Yes | V | M | T | | S | | Y | | S | |

| | | | | | | | | | | | |
|---|---|---|---|---|---|---|---|---|---|---|---|
| ¹ Single amino acid letters indicate the amino acid substitution of the corresponding amino acid relative to the wildtype PBP2 sequence when optimally aligned and using the amino acid numbering of the wildtype sequence. "Yes" for mosaicism indicates an amino acid sequence other than GAE at amino acid position 375-377 relative to the wildtype PBP2 sequence when optimally aligned and using the amino acid numbering of the wildtype sequence. | | | | | | | | | | | |

### Estimation of Assay Sensitivity

A parsimonious group of *pen*A amino acid locations to predict decreased susceptibility to cefixime in *N. gonorrhoeae* strains was proposed. The sensitivity of a hypothetical assay for predicting decreased susceptibility using those locations was estimated by calculating the number of isolates with genotypic mutations in those locations divided by the number of phenotypically decreased susceptible isolates using the data summarized in Table 1.

### Molecular Mechanisms of Cefixime Decreased Susceptibility

Table 3 shows a list of *pen*A types from the NG-STAR database and the presence or absence of specific gene alterations and associated amino acid changes that predict cefixime decreased susceptibility. Decreased susceptibility to cefixime has been associated with many genetic alterations in *the penA* gene. There is strong laboratory and epidemiological research supporting mosaicism and other point mutations of the *penA* gene as mechanisms for cefixime decreased susceptibility by means of PBP2 target alteration. Evidence supporting the importance of alterations in other genes are not as compelling. However, there is evidence that isolates with identical *penA* alleles could have different MIC levels (susceptible with MIC < 0.125 µg/mL versus highly resistant with MIC ≥ 0.5 µg/mL), indicating the involvement of other genes in decreasing susceptibility to cefixime. Several candidate genes impacting decreased *N. gonorrhoeae* susceptibility include *mtr*R*,* the transcriptional regulator for the *Mtr*CDE efflux pump system; *pon*A*,* encoding for penicillin binding protein 1 (PBP1); *pil*Q*,* encoding for the type IV pilus secretin; and *pen*B (alias: *por*B1b), encoding for a major outer membrane protein *por*B, a porin.

### penA - penA mosaicism

Mosaicism of the *pen*A gene in *N. gonorrhoeae* was first described by Ameyama *et al.,* who found that some *pen*A nucleotide sequences of *N. gonorrhoeae* contained portions that highly resemble those of other non-pathogenic or commensal *Neisseria* species, such as *N. perflava*, *N. cinerea, N. flavescens*, and *N. meningitidis.* As used herein, *penA* mosaicism refers to a *pen*A gene of a given *Neisseria* species that comprises a multitude of nucleotide and amino acid changes thought to be acquired from *the penA* gene of another by transformation or conjugation and the spontaneous generation of mutations. *See, e.g.,* US 9,297,048.

Mosaicism in *pen*A was found to be the primary determinant of cefixime decreased susceptibility by many studies. From an aggregate of 415 *N. gonorrhoeae* isolates with an MIC ≥ 0.12 µg/mL from 25 reports representing 22 countries, 83.1% (345 out of 415) were found to have *mosaic pen*A genes (Table 1). Among all the different mosaic patterns, *pen*A10 and *pen*A34 were the most frequently reported mosaic *penA* patterns of all isolates with mosaic *penA* gene mutations among *N. gonorrhoeae* strains with reduced cefixime susceptibility (accounting for 39.1% (135 out of 345) and 49.9% (172 out of 345), respectively). While *pen*A34 was found worldwide, *pen*A10 was mostly found in Asia, and was also associated with resistance to, and treatment failure by, another third-generation cephalosporin, ceftibuten.

1312M, V316T, N512Y, and G545S amino acid substitutions are frequently seen in mosaic *penA* patterns. Researchers have found that amino acid substitutions I312M, V316T/P, and G545 are associated with reduced cefixime susceptibility. However, a gene transformation study showed that when introduced into a wild-type *pen*A, I312M, V316T and G545S together only minimally elevated the cefixime MIC. The reversion of the triple mutation in a cefixime-resistant strain 35/02 (with *pen*A10) back into wild-type returned its MIC to that similar to the wild-type *penA* MIC, indicating that these three mutations are important to cefixime resistance only in the context of other mutations found in mosaic *pen*A10 alleles.

Chimeric *penA* genes were created by replacing sequential portions of *pen* A10 by the corresponding regions of a wild-type *penA* gene. Reversion of amino acid regions 309-353 (containing I312M and V316T and 13 other substitutions), 489-528 (containing N512Y and 2 other mutations) and 528-581 (containing G545S and 9 other substitutions), showed significant decrease in MIC. Among the three, the reversion of region 528-581 decreased the MIC to such a significant degree that the chimera could not be selected despite multiple attempts, indicating mutations in this region may be critical factors in influencing cefixime susceptibility. When the G545S substitution was re-introduced to the 528-581-wild-type *pen*A10*,* the resulting strain's cefixime MIC only rose to 0.05 µg/mL, suggesting that other mutations in the 528-581 region were necessary to significantly elevate the cefixime MIC to > 0.125 µg/mL.

The reversion of region 489-528 (containing N512Y and 2 other mutations) decreased the MIC from about 0.125 µg/mL to about 0.05 µg/mL. Y512N reversion alone presented a decreased MIC from about 0.125 µg/mL to about 0.06 µg/mL. Accounting for most of the effect on cefixime resistance by mutations in the 489-528 region, N512Y showed major importance in conferring cefixime resistance in the context of other mosaic changes.

In summary, I312M, V316T, N512Y, and G545S were found to be important to cefixime decreased susceptibility or resistance only in the context of other mutations found in mosaic *penA* alterations. All of them were associated with but none was necessary or sufficient for cefixime decreased susceptibility or resistance.

### penA - non-mosaic penA with point amino acid alterations: A501 + G542 + P551

Although the penA34 mosaicism was present in 98% of all isolates with an MIC ≥ 0.25 µg/mL in a study on more than 1100 *N. gonorrhoeae* isolates collected in the United States, the percentage lowered to 91% when a lower, and more clinically relevant MIC breakpoint (≥ 0.125 µg/mL), was used. That indicates the role of other non-*pen*A34 mutations, most notably, other amino acid substitutions in *the penA* gene. In the last decade, more than a dozen cefixime-resistant *N. gonorrhoeae* strains reported in Asia and Europe were found to have non-mosaic *pen*A alleles (Table 1). Among reports from Asia, over 20% of *N. gonorrhoeae* strains with decreased susceptibility to cefixime had non-mosaic *penA* mutations. Gene transformation experiments have identified multiple, important amino acid substitutions in non-mosaic *pen*A that significantly decrease cefixime susceptibility.

Three amino acid substitutions, A501 S/V/T/P, G542S, and P551 S/L/P, have been associated with cefixime decreased susceptibility independent from *pen*A mosaicism. An independent A501 substitution potentially decreased cefixime susceptibility by increasing the rigidity of PBP2 active site.

Table 1 summarizes the cefixime decreased susceptible related *penA* amino acid alterations in all *N. gonorrhoeae* strains with cefixime MIC ≥ 0.12 µg/mL. One important finding is that 68 out of 70 (97.1%) non-mosaic *pen*A strains with reduced susceptibility to cefixime have point mutation in at least one of the three codons, A501, G542, and P551. Additionally, decreased susceptible *N. gonorrhoeae* strains with non-mosaic *penA* have mostly been reported in Asia and Europe.

Figure 1 summarizes the MIC levels of all *N. gonorrhoeae* strains with cefixime MIC ≥ 0.12 µg/mL by different combinations of decreased susceptible related *penA* amino acid alterations (n = 240). Strains lacking specific cefixime MIC value or *pen*A alteration records (n = 175) were excluded.

### mtrR

*mtrR* is a repressor gene that regulates the expression of the MtrCDE efflux pump system, an important mechanism in transporting antimicrobial agents out of the bacterial cell. Changes in the promoter or coding sequence of the *mtrR* gene can potentially decrease antimicrobial susceptibility by increased efflux. There are conflicting reports on the importance of the *mtrR* gene in cefixime decreased susceptibility. Mutations frequently found in strains with cefixime decreased susceptibility include a -35A deletion in the promoter region, plus A39T and G45D in the coding region. No reports about gene transformation studies looking at the contributions of *mtr*R alterations to cefixime resistance independent from *pen*A changes were found in the art. Nonetheless, other studies report that mutations in *mtrR* gene have little or no association with cefixime susceptibility.

### penB (porB1b)

*pen*B, also known as *por*B1b, encodes for an outer membrane porin and is thought to increase penicillin resistance by changing the bacterial membrane permeability to certain antibiotics when *pen*A and *mtrR* mutations are also present, although its role in cefixime resistance is unclear. Alterations found in strains with cefixime decreased susceptibility include G120K and A121N/D substitutions. While a study in the United States suggested no correlation between cefixime decreased susceptibility and G120K or G120D/A121D, the lack of G120K *and* A121N mutations strongly predicted susceptibility.

Two other mutations in *penB* gene commonly found in cefixime decreased susceptible strains are G101K/D and A102D/N/S. Combination of mutations at those 2 sites were found in 175 *N. gonorrhoeae* strains having decreased susceptibility to cefixime.

No evidence that alterations in *pen*B gene alone can confer decreased susceptibility to cefixime was found in the prior art.

### ponA

*ponA* encodes for penicillin binding protein 1 (PBP1), an additional cell wall protein important in beta-lactam antibiotic antimicrobial activity. Alterations in *pon*A gene were associated with penicillin resistance by PBP1 target mutation, although its role in cefixime decreased susceptibility is unclear. One mutation, L421P, was frequently found in cefixime decreased susceptible strains, but a gene transformation study showed that a *pon*A L421P substitution does not contribute additional decreased susceptibility to cefixime without a mosaic *pen*A gene.

### pilQ

*pilQ* encodes for a type IV pili secretin. Mutations in the *pil*Q gene are thought to increase penicillin resistance by changing the bacterial membrane permeability when *pen*A, *mtr*R or *pen*B mutations are also present, although its role in cefixime resistance is also unclear.

While one study concluded that changes in the *pil*Q gene are unlikely associated with cefixime decreased susceptibility, another study found that a 176-183 deletion (vs. full length), N341S, D526N/G, or N648S each strongly predicted *N. gonorrhoeae* susceptibility to cefixime. Notably, among those four mutations, N648S was the only mutation that was found to be relatively common (in 23.7% of all isolates compared to ≤ 10% for any of the other three).

### REFERENCES

1. Unemo M, Shafer WM. Antibiotic resistance in Neisseria gonorrhoeae: origin, evolution, and lessons learned for the future. Annals of the New York Academy of Sciences. 2011;1230:E19-E28.
2. Tapsall JW, Ndowa F, Lewis DA, Unemo M. Meeting the public health challenge of multidrug- and extensively drug-resistant Neisseria gonorrhoeae. Expert Rev Anti Infect Ther. 2009;7(7):821-834.
3. Tanvir SB, Qasim SSB, Shariq A, Najeeb S, Shah AH. Systematic review and meta-analysis on efficacy of cefixime for treating gonococcal infections. International journal of health sciences. 2018;12(5):90.
4. Unemo M, Lahra MM, Cole M, et al. The WHO Global Gonococcal Antimicrobial Surveillance Programme (67 surveyed countries in 2015-2016) - an observational study emphasising essential global actions. Sexual Health. In press, 2019.
5. Organization WH. WHO guidelines for the treatment of Neisseria gonorrhoeae. World Health Organization; 2016.
6. Workowski KA, Bolan GA. Sexually transmitted diseases treatment guidelines, 2015. MMWR Recommendations and reports: Morbidity and mortality weekly report Recommendations and reports. 2015;64(RR-03):1.
7. Fifer H, Saunders J, Suneeta S, Sadiq ST, FitzGerald M. British Association for Sexual Health and HIV national guideline for the management of infection with Neisseria gonorrhoeae (2019). 2019.
8. Olsen B, Pham TL, Golparian D, Johansson E, Tran HK, Unemo M. Antimicrobial susceptibility and genetic characteristics of Neisseria gonorrhoeae isolates from Vietnam, 2011. BMC infectious diseases. 2013; 13:40.
9. Unemo M, Golparian D, Nicholas R, Ohnishi M, Gallay A, Sednaoui P. High-level cefixime- and ceftriaxone-resistant Neisseria gonorrhoeae in France: novel penA mosaic allele in a successful international clone causes treatment failure. Antimicrobial agents and chemotherapy. 2012;56(3): 1273-1280.
10. Lee H, Unemo M, Kim HJ, Seo Y, Lee K, Chong Y. Emergence of decreased susceptibility and resistance to extended-spectrum cephalosporins in Neisseria gonorrhoeae in Korea. The Journal of antimicrobial chemotherapy. 2015;70(9):2536-2542.
11. Ohnishi M, Watanabe Y, Ono E, et al. Spread of a chromosomal cefixime-resistant penA gene among different Neisseria gonorrhoeae lineages. Antimicrobial agents and chemotherapy. 2010;54(3):1060-1067.
12. Morita-Ishihara T, Unemo M, Furubayashi K, et al. Treatment failure with 2 g of azithromycin (extended-release formulation) in gonorrhoea in Japan caused by the international multidrug-resistant ST1407 strain of Neisseria gonorrhoeae. The Journal of antimicrobial chemotherapy. 2014;69(8):2086-2090.
13. Ryan L, Golparian D, Fennelly N, et al. Antimicrobial resistance and molecular epidemiology using whole-genome sequencing of Neisseria gonorrhoeae in Ireland, 2014-2016: focus on extended-spectrum cephalosporins and azithromycin. European journal of clinical microbiology & infectious diseases : official publication of the European Society of Clinical Microbiology. 2018.
14. Ito M, Deguchi T, Mizutani KS, et al. Emergence and spread of Neisseria gonorrhoeae clinical isolates harboring mosaic-like structure of penicillin-binding protein 2 in Central Japan. Antimicrobial agents and chemotherapy. 2005;49(1):137-143.
15. Gianecini R, Romero MLM, Oviedo C, Vacchino M, Galarza P. Emergence and Spread of Neisseria gonorrhoeae Isolates With Decreased Susceptibility to Extended-Spectrum Cephalosporins in Argentina, 2009 to 2013. Sexually transmitted diseases. 2017;44(6):351-355.
16. Martin I, Sawatzky P, Allen V, et al. Emergence and characterization of Neisseria gonorrhoeae isolates with decreased susceptibilities to ceftriaxone and cefixime in Canada: 2001-2010. Sexually transmitted diseases. 2012;39(4):316-323.
17. Serra-Pladevall J, Barbera MJ, Rodriguez S, et al. Neisseria gonorrhoeae antimicrobial susceptibility in Barcelona: penA, ponA, mtrR, and porB mutations and NG-MAST sequence types associated with decreased susceptibility to cephalosporins. European journal of clinical microbiology & infectious diseases : official publication of the European Society of Clinical Microbiology. 2016;35(9):1549-1556.
18. Jiang FX, Lan Q, Le WJ, Su XH. Antimicrobial susceptibility of Neisseria gonorrhoeae isolates from Hefei (2014-2015): genetic characteristics of antimicrobial resistance. BMC infectious diseases. 2017;17(1):366.
19. Takahata S, Senju N, Osaki Y, Yoshida T, Ida T. Amino acid substitutions in mosaic penicillin-binding protein 2 associated with reduced susceptibility to cefixime in clinical isolates of Neisseria gonorrhoeae. Antimicrobial agents and chemotherapy. 2006;50(11):3638-3645.
20. Allen VG, Farrell DJ, Rebbapragada A, et al. Molecular analysis of antimicrobial resistance mechanisms in Neisseria gonorrhoeae isolates from Ontario, Canada. Antimicrobial agents and chemotherapy. 2011;55(2):703-712.
21. Carannante A, Vacca P, Ghisetti V, et al. Genetic Resistance Determinants for Cefixime and Molecular Analysis of Gonococci Isolated in Italy. Microbial drug resistance (Larchmont, NY). 2017;23(2):247-252.
22. Jeverica S, Golparian D, Maticic M, Potocnik M, Mlakar B, Unemo M. Phenotypic and molecular characterization of Neisseria gonorrhoeae isolates from Slovenia, 2006-12: rise and fall of the multidrug-resistant NG-MAST genogroup 1407 clone? The Journal of antimicrobial chemotherapy. 2014;69(6):1517-1525.
23. Endimiani A, Guilarte YN, Tinguely R, et al. Characterization of Neisseria gonorrhoeae isolates detected in Switzerland (1998-2012): emergence of multidrug-resistant clones less susceptible to cephalosporins. BMC infectious diseases. 2014;14:106.
24. Allen VG, Mitterni L, Seah C, et al. Neisseria gonorrhoeae treatment failure and susceptibility to cefixime in Toronto, Canada. Jama. 2013;309(2):163-170.
25. Pandori M, Barry PM, Wu A, et al. Mosaic penicillin-binding protein 2 in Neisseria gonorrhoeae isolates collected in 2008 in San Francisco, California. Antimicrobial agents and chemotherapy. 2009;53(9):4032-4034.
26. Camara J, Serra J, Ayats J, et al. Molecular characterization of two high-level ceftriaxone-resistant Neisseria gonorrhoeae isolates detected in Catalonia, Spain. The Journal of antimicrobial chemotherapy. 2012;67(8): 1858-1860.
27. Grad YH, Harris SR, Kirkcaldy RD, et al. Genomic Epidemiology of Gonococcal Resistance to Extended-Spectrum Cephalosporins, Macrolides, and Fluoroquinolones in the United States, 2000-2013. The Journal of infectious diseases. 2016;214(10):1579-1587.
28. Lewis DA, Sriruttan C, Muller EE, et al. Phenotypic and genetic characterization of the first two cases of extended-spectrum-cephalosporin-resistant Neisseria gonorrhoeae infection in South Africa and association with cefixime treatment failure. The Journal of antimicrobial chemotherapy. 2013;68(6): 1267-1270.
29. Washington MA, Jerse AE, Rahman N, et al. First description of a cefixime- and ciprofloxacin-resistant Neisseria gonorrhoeae isolate with mutations in key antimicrobial susceptibility-determining genes from the country of Georgia. New microbes and new infections. 2018;24:47-51.
30. Lahra MM, Martin I, Demczuk W, et al. Cooperative Recognition of Internationally Disseminated Ceftriaxone-Resistant Neisseria gonorrhoeae Strain. Emerging infectious diseases. 2018;24(4).
31. Yokoi S, Deguchi T, Ozawa T, et al. Threat to cefixime treatment for gonorrhea. Emerging infectious diseases. 2007;13(8):1275-1277.
32. Unemo M, Golparian D, Syversen G, Vestrheim DF, Moi H. Two cases of verified clinical failures using internationally recommended first-line cefixime for gonorrhoea treatment, Norway, 2010. Euro surveillance : bulletin Europeen sur les maladies transmissibles = European communicable disease bulletin. 2010;15(47).
33. Ison CA, Hussey J, Sankar KN, Evans J, Alexander S. Gonorrhoea treatment failures to cefixime and azithromycin in England, 2010. Euro surveillance : bulletin Europeen sur les maladies transmissibles = European communicable disease bulletin. 2011; 16(14).
34. Forsyth S, Penney P, Rooney G. Cefixime-resistant Neisseria gonorrhoeae in the UK: a time to reflect on practice and recommendations. Int J STD AIDS. 2011;22(5):296-297.
35. Unemo M, Golparian D, Stary A, Eigentler A. First Neisseria gonorrhoeae strain with resistance to cefixime causing gonorrhoea treatment failure in Austria, 2011. Euro surveillance : bulletin Europeen sur les maladies transmissibles = European communicable disease bulletin. 2011;16(43).
36. Cristillo AD, Bristow CC, Torrone E, et al. Antimicrobial Resistance in Neisseria gonorrhoeae: Proceedings of the STAR Sexually Transmitted Infection-Clinical Trial Group Programmatic Meeting. Sexually transmitted diseases. 2018.
37. Barry PM, Klausner JD. The use of cephalosporins for gonorrhea: the impending problem of resistance. Expert Opin Pharmacother. 2009;10(4):555-577.
38. Blank S, Daskalakis DC. Neisseria gonorrhoeae - Rising Infection Rates, Dwindling Treatment Options. New England Journal of Medicine. 2018;379(19):1795-1797.
39. Dougherty TJ, Koller AE, Tomasz A. Penicillin-binding proteins of penicillin-susceptible and intrinsically resistant Neisseria gonorrhoeae. Antimicrobial agents and chemotherapy. 1980;18(5):730-737.
40. Ameyama S, Onodera S, Takahata M, et al. Mosaic-like structure of penicillin-binding protein 2 Gene (penA) in clinical isolates of Neisseria gonorrhoeae with reduced susceptibility to cefixime. Antimicrobial agents and chemotherapy. 2002;46(12):3744-3749.
41. Gose S, Nguyen D, Lowenberg D, Samuel M, Bauer H, Pandori M. Neisseria gonorrhoeae and extended-spectrum cephalosporins in California: surveillance and molecular detection of mosaic penA. BMC infectious diseases. 2013;13:570.
42. Chen CC, Yen MY, Wong WW, et al. Tracing subsequent dissemination of a cluster of gonococcal infections caused by an ST1407-related clone harbouring mosaic penA alleles in Taiwan. The Journal of antimicrobial chemotherapy. 2013;68(7):1567-1571.
43. Ochiai S, Ishiko H, Yasuda M, Deguchi T. Rapid detection of the mosaic structure of the Neisseria gonorrhoeae penA Gene, which is associated with decreased susceptibilities to oral cephalosporins. Journal of clinical microbiology. 2008;46(5):1804-1810.
44. EUCAST. Breakpoint tables for interpretation of MICs and zone diameters. Version 8.1. http://wwweucastorg. 2018.
45. CLSI. Performance Standards for Antimicrobial Susceptibility Testing. 28th ed. CLSI supplement M100. 2018.
46. Ohnishi M, Golparian D, Shimuta K, et al. Is Neisseria gonorrhoeae Initiating a Future Era of Untreatable Gonorrhea?: Detailed Characterization of the First Strain with High-Level Resistance to Ceftriaxone. Antimicrobial agents and chemotherapy. 2011;55(7):3538.
47. Demczuk W, Sidhu S, Unemo M, et al. Neisseria gonorrhoeae Sequence Typing for Antimicrobial Resistance, a Novel Antimicrobial Resistance Multilocus Typing Scheme for Tracking Global Dissemination of N. gonorrhoeae Strains. Journal of clinical microbiology. 2017;55(5):1454-1468.
48. Ameyama S, Onodera S, Takahata M, et al. Mosaic-Like Structure of Penicillin-Binding Protein 2 Gene (penA) in Clinical Isolates of Neisseria gonorrhoeae with Reduced Susceptibility to Cefixime. Antimicrobial agents and chemotherapy. 2002;46(12):3744-3749.
49. Tomberg J, Unemo M, Davies C, Nicholas RA. Molecular and structural analysis of mosaic variants of penicillin-binding protein 2 conferring decreased susceptibility to expanded-spectrum cephalosporins in Neisseria gonorrhoeae: role of epistatic mutations. Biochemistry. 2010;49(37):8062-8070.
50. Tomberg J, Unemo M, Ohnishi M, Davies C, Nicholas RA. Identification of amino acids conferring high-level resistance to expanded-spectrum cephalosporins in the penA gene from Neisseria gonorrhoeae strain H041. Antimicrobial agents and chemotherapy. 2013;57(7):3029-3036.
51. Tomberg J, Fedarovich A, Vincent LR, et al. Alanine 501 Mutations in Penicillin-Binding Protein 2 from Neisseria gonorrhoeae: Structure, Mechanism, and Effects on Cephalosporin Resistance and Biological Fitness. Biochemistry. 2017;56(8):1140-1150.
52. Pan W, Spratt BG. Regulation of the permeability of the gonococcal cell envelope by the mtr system. Molecular microbiology. 1994; 11(4):769-775.
53. Ropp PA, Hu M, Olesky M, Nicholas RA. Mutations in ponA, the gene encoding penicillin-binding protein 1, and a novel locus, penC, are required for high-level chromosomally mediated penicillin resistance in Neisseria gonorrhoeae. Antimicrobial agents and chemotherapy. 2002;46(3):769-777.
54. Zhao S, Tobiason DM, Hu M, Seifert HS, Nicholas RA. The penC mutation conferring antibiotic resistance in Neisseria gonorrhoeae arises from a mutation in the PilQ secretin that interferes with multimer stability. Molecular microbiology. 2005;57(5):1238-1251.
55. Olesky M, Zhao S, Rosenberg RL, Nicholas RA. Porin-mediated antibiotic resistance in Neisseria gonorrhoeae: ion, solute, and antibiotic permeation through PIB proteins with penB mutations. Journal of bacteriology. 2006;188(7):2300-2308.
56. Harris SR, Cole MJ, Spiteri G, et al. Public health surveillance of multidrug-resistant clones of Neisseria gonorrhoeae in Europe: a genomic survey. The Lancet Infectious Diseases. 2018;18(7):758-768.
57. Shimuta K, Watanabe Y, Nakayama S, et al. Emergence and evolution of internationally disseminated cephalosporin-resistant Neisseria gonorrhoeae clones from 1995 to 2005 in Japan. BMC infectious diseases. 2015;15:378.
58. Grad YH, Kirkcaldy RD, Trees D, et al. Genomic epidemiology of Neisseria gonorrhoeae with reduced susceptibility to cefixime in the USA: a retrospective observational study. The Lancet Infectious diseases. 2014;14(3):220-226.
59. Lo JY, Ho KM, Leung AO, et al. Ceftibuten resistance and treatment failure of Neisseria gonorrhoeae infection. Antimicrobial agents and chemotherapy. 2008;52(10):3564-3567.
60. Unemo M, Nicholas, R. A., Jerse, A. E., Davies, C., and Shafer, W. M. Molecular mechanisms of antibiotic resistance expressed by the pathogenic Neisseria. In: Pathogenic Neisseria. 2nd ed. Norfolk, U.K.: Caister Academic Press; 2014.
61. Dona V, Kasraian S, Lupo A, et al. Multiplex Real-Time PCR Assay with High-Resolution Melting Analysis for Characterization of Antimicrobial Resistance in Neisseria gonorrhoeae. Journal of clinical microbiology. 2016;54(8):2074-2081.
62. Zhao S, Duncan M, Tomberg J, Davies C, Unemo M, Nicholas RA. Genetics of chromosomally mediated intermediate resistance to ceftriaxone and cefixime in Neisseria gonorrhoeae. Antimicrobial agents and chemotherapy. 2009;53(9):3744-3751.
63. Whiley DM, Jacobsson S, Tapsall JW, Nissen MD, Sloots TP, Unemo M. Alterations of the pilQ gene in Neisseria gonorrhoeae are unlikely contributors to decreased susceptibility to ceftriaxone and cefixime in clinical gonococcal strains. The Journal of antimicrobial chemotherapy. 2010;65(12):2543-2547.
64. Lee SG, Lee H, Jeong SH, et al. Various penA mutations together with mtrR, porB and ponA mutations in Neisseria gonorrhoeae isolates with reduced susceptibility to cefixime or ceftriaxone. The Journal of antimicrobial chemotherapy. 2010;65(4):669-675.
65. Organization WH. Global action plan on antimicrobial resistance. 2015. In:2017.
66. Buono SA, Watson TD, Borenstein LA, Klausner JD, Pandori MW, Godwin HA. Stemming the tide of drug-resistant Neisseria gonorrhoeae: the need for an individualized approach to treatment. Journal of Antimicrobial Chemotherapy. 2015;70(2):374-381.
67. Klausner JD, Kerndt P. Cephalosporin resistance in neisseria gonorrhoeae infections. Jama. 2013;309(19):1989-1991.
68. Wong LK HP, Soge OO, Humphries RM, Klausner JD. Real-time PCR targeting the penA mosaic XXXIV type for prediction of extended-spectrum- cephalosporin susceptibility in clinical Neisseria gonorrhoeae isolates. Antimicrobial agents and chemotherapy. 2017;61(11).
69. Lo JY, Ho KM, Lo AC. Surveillance of gonococcal antimicrobial susceptibility resulting in early detection of emerging resistance. The Journal of antimicrobial chemotherapy. 2012;67(6): 1422-1426.
70. Shimuta K, Unemo M, Nakayama S, et al. Antimicrobial resistance and molecular typing of Neisseria gonorrhoeae isolates in Kyoto and Osaka, Japan, 2010 to 2012: intensified surveillance after identification of the first strain (H041) with high-level ceftriaxone resistance. Antimicrobial agents and chemotherapy. 2013;57(11):5225-5232.

All scientific and technical terms used in this application have meanings commonly used in the art unless otherwise specified.

As used herein, "cefixime compounds" refer to compounds having the following structural formula (I) as part of its backbone structure: 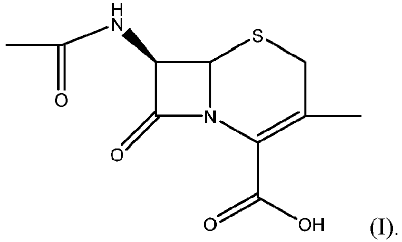 Exemplary cefixime compounds include Cefacetrile, Cefaclor, Cefadroxil, Cefalexin, Cefaloglycin, Cefalonium, Cefaloram, Cefaloridine, Cefalotin, Cefaparole, Cefapirin, Cefatrizine, Cefazaflur, Cefazedone, Cefazolin, Cefbuperazone, Cefcanel, Cefcapene, Cefclidine, Cefdaloxime, Cefdinir, Cefditoren, Cefedrolor, Cefempidone, Cefepime, Cefetamet, Cefetrizole, Cefivitril, Cefixime, Cefluprenam, Cefmatilen, Cefmenoxime, Cefmepidium, Cefmetazole, Cefmetazole, Cefminox, Cefodizime, Cefonicid, Cefoperazone, Cefoselis, Cefotaxime, Cefotetan, Cefotetan, Cefotiam, Cefovecin, Cefoxazole, Cefoxitin, Cefoxitin, Cefozopran, Cefpimizole, Cefpirome, Cefpodoxime, Cefprozil, Cefquinome, Cefradine, Cefrotil, Cefroxadine, Cefsumide, Ceftamere, Ceftaroline, Ceftazidime, Cefteram, Ceftezole, Ceftibuten, Ceftiofur, Ceftiolene, Ceftioxide, Ceftizoxime, Ceftobiprole, Ceftolozane, Cefuracetime, Cefuroxime, Cefuzonam, Cephamycin, Flomoxef, Latamoxef, Loracarbef, Nitrocefin, Oxacephems, and the like. In some embodiments, the cefixime compounds are third generation cephalosporins such as Cefcapene, Cefdaloxime, Cefdinir, Cefditoren, Cefetamet, Cefixime, Cefmenoxime, Cefodizime, Cefoperazone, Cefotaxime, Cefovecin, Cefpimizole, Cefpodoxime, Ceftamere, Ceftazidime, Cefteram, Ceftibuten, Ceftiofur, Ceftiolene, Ceftizoxime, Ceftriaxone, and Latamoxef. In some embodiments, the cefixime compound is Cefixime or Ceftriaxone, preferably Cefixime. Thus, antibacterials that are not cefixime compounds lack structural formula (I) as part of its backbone structure, such antibacterials included Sulfonamides, Tetracyclines, Aminoglycosides, Macrolides, Ketolides, Quinolones, Lincomycins, and Glycopeptides.

As used herein, the terms "subject", "patient", and "individual" are used interchangeably to refer to humans and non-human animals. The term "non-human animal" includes all vertebrates, *e.g*., mammals and non-mammals, such as non-human primates, horses, sheep, dogs, cows, pigs, chickens, and other veterinary subjects and test animals. In some embodiments, the subject is a mammal. In some embodiments, the subject is a human.

As used herein, the term "diagnosing" refers to the physical and active step of informing, *i.e.,* communicating verbally or by writing (on, *e.g.,* paper or electronic media), another party, *e.g.,* a patient, of the diagnosis. Similarly, "providing a prognosis" refers to the physical and active step of informing, *i.e.,* communicating verbally or by writing (on, *e.g.,* paper or electronic media), another party, *e.g.,* a patient, of the prognosis.

The use of the singular can include the plural unless specifically stated otherwise. As used in the specification and the appended claims, the singular forms "a", "an", and "the" can include plural referents unless the context clearly dictates otherwise.

As used herein, "and/or" means "and" or "or". For example, "A and/or B" means "A, B, or both A and B" and "A, B, C, and/or D" means "A, B, C, D, or a combination thereof" and said "A, B, C, D, or a combination thereof" means any subset of A, B, C, and D, for example, a single member subset (*e.g.,* A or B or C or D), a two-member subset (*e.g.,* A and B; A and C; *etc.*), or a three-member subset (*e.g.*, A, B, and C; or A, B, and D; *etc.*), or all four members (*e.g.*, A, B, C, and D).

As used herein, the phrase "one or more of", *e.g.,* "one or more of A, B, and/or C" means "one or more of A", "one or more of B", "one or more of C", "one or more of A and one or more of B", "one or more of B and one or more of C", "one or more of A and one or more of C" and "one or more of A, one or more of B, and one or more of C".

Similarly, a sentence reciting a string of alternates is to be interpreted as if a string of sentences were provided such that each given alternate was provided in a sentence by itself. For example, the sentence "In some embodiments, the composition comprises A, B, or C" is to be interpreted as if written as the following three separate sentences: "In some embodiments, the composition comprises A. In some embodiments, the composition comprises B. In some embodiments, the composition comprises C." As another example, the sentence "In some embodiments, the composition comprises at least A, B, or C" is to be interpreted as if written as the following three separate sentences: "In some embodiments, the composition comprises at least A. In some embodiments, the composition comprises at least B. In some embodiments, the composition comprises at least C."

Having thus described exemplary embodiments of the present invention, it should be noted by those skilled in the art that the within disclosures are exemplary only and that various other alternatives, adaptations, and modifications may be made within the scope of the present invention. Accordingly, the present invention is not limited to the specific embodiments as illustrated herein, but is only limited by the following claims.

## Claims

1. An in vitro method for inhibiting the growth of a given *Neisseria* species, which comprises
(A) determining the presence or absence of amino acid alterations at amino acid positions 345, 375-377, 501, 542, and 551 in the penicillin-binding protein 2 (PBP2) of the given *Neisseria* species;
(B) characterizing the given *Neisseria* species as being susceptible to cefixime compounds where amino acid alterations at amino acid positions 345, 375-377, 501, 542, and 551 are absent; and
(C) contacting the given *Neisseria* species with a cefixime compound where the given *Neisseria* species is characterized as being susceptible to cefixime compounds, and contacting the given *Neisseria* species with an antibacterial other than cefixime compounds where the given *Neisseria* species is characterized as being resistant to cefixime compounds.

2. An in vitro method for characterizing whether a given *Neisseria* species is susceptible to a cefixime compound, which comprises
(A) determining the presence or absence of amino acid alterations at amino acid positions 345, 375-377, 501, 542, and 551 of its penicillin-binding protein 2 (PBP2), and
(B) characterizing the given *Neisseria* species as being susceptible to the cefixime compound where amino acid alterations at amino acid positions 345, 375-377, 501, 542, and 551 are absent.

3. The method according to any one of claims 1 and 2, wherein the given *Neisseria* species is a *Neisseria* gonorrhoeae strain.

4. The method according to any one of claims 1 to 3, wherein the cefixime compound is cefixime.

5. The method according to any one of claims 1, 3 and 4, wherein the antibacterial other than cefixime compounds is selected from the following groups of antibiotics: Sulfonamides, Tetracyclines, Aminoglycosides, Macrolides, Ketolides, Quinolones, Lincomycins, and Glycopeptides.

## Patentansprüche

1. In-vitro-Verfahren zum Hemmen des Wachstums einer gegebenen Neisseria-Spezies, das umfasst
(A) Bestimmen der Anwesenheit oder Abwesenheit von Aminosäureveränderungen an den Aminosäurepositionen 345, 375-377, 501, 542 und 551 in dem Penicillin-bindenden Protein 2 (PBP2) der gegebenen *Neisseria-*Spezies;
(B) Charakterisieren der gegebenen Neisseria-Spezies als empfindlich gegenüber Cefiximverbindungen sind, wobei Aminosäureveränderungen an den Aminosäurepositionen 345, 375-377, 501, 542 und 551 abwesend sind; und
(C) Inkontaktbringen der gegebenen *Neisseria*-Spezies mit einer Cefiximverbindung, wobei die gegebene *Neisseria*-Spezies **gekennzeichnet ist als** empfindlich gegen Cefiximverbindungen zu sein, und Inkontaktbringen der gegebenen *Neisseria*-Spezies mit einer anderen antibakteriellen Verbindung als Cefiximverbindungen, wobei die gegebenen *Neisseria*-Spezies **gekennzeichnet ist als** resistent gegen Cefiximverbindungen zu sein.

2. In-vitro-Verfahren zum Charakterisieren, ob eine gegebene *Neisseria-*Spezies empfindlich gegenüber einer Cefiximverbindung ist, das umfasst
(A) Bestimmen der Anwesenheit oder Abwesenheit von Aminosäureveränderungen an den Aminosäurepositionen 345, 375-377, 501, 542 und 551 seines Penicillin-bindenden Proteins 2 (PBP2) und
(B) Charakterisieren der gegebenen *Neisseria*-Spezies als empfindlich gegenüber der Cefiximverbindung, wobei Aminosäureveränderungen an den Aminosäurepositionen 345, 375-377, 501, 542 und 551 abwesend sind.

3. Verfahren nach einem der Ansprüche 1 und 2, wobei die gegebene *Neisseria*-Spezies ein *Neisseria*-gonorrhoeae-Stamm ist.

4. Verfahren nach einem der Ansprüche 1 bis 3, wobei die Cefiximverbindung Cefixim ist.

5. Verfahren nach einem der Ansprüche 1, 3 und 4, wobei die anderen antibakteriellen Verbindungen als Cefixim aus den folgenden Antibiotikagruppen ausgewählt sind: Sulfonamide, Tetracycline, Aminoglykoside, Makrolide, Ketolide, Chinolone, Lincomycine und Glykopeptide.

## Revendications

1. Procédé in vitro destiné à inhiber la croissance d'une espèce donnée de *Neisseria,* qui comprend
(A) la détermination de la présence ou de l'absence d'altérations des acides aminés aux positions 345, 375 à 377, 501, 542 et 551 de la protéine 2 de liaison à la pénicilline (PBP2) de l'espèce *donnée de Neisseria donnée* ;
(B) la caractérisation de l'espèce donnée de *Neisseria* comme étant sensible aux composés de céfixime en l'absence d'altérations des acides aminés aux positions 345, 375 à 377, 501, 542 et 551 ; et
(C) la mise en contact de l'espèce donnée de *Neisseria* avec un composé de céfixime lorsque l'espèce donnée de *Neisseria* est **caractérisée comme** étant sensible aux composés de céfixime, et la mise en contact de l'espèce donnée de *Neisseria* avec un antibactérien autre que les composés de céfixime lorsque l'espèce donnée de *Neisseria* est **caractérisée comme** étant résistante aux composés de céfixime.

2. Procédé in vitro destiné à déterminer le fait de savoir si une espèce donnée de *Neisseria* est sensible à un composé de céfixime, qui comprend
(A) la détermination de la présence ou de l'absence d'altérations des acides aminés aux positions 345, 375 à 377, 501, 542 et 551 de sa protéine 2 de liaison à la pénicilline (PBP2), et
(B) la caractérisation de l'espèce donnée de *Neisseria* comme étant sensible au composé de céfixime en l'absence d'altérations des acides aminés aux positions 345, 375 à 377, 501, 542 et 551.

3. Procédé selon l'une quelconque des revendications 1 et 2, dans lequel l'espèce donnée de *Neisseria* est une souche de *Neisseria* gonorrhoeae.

4. Procédé selon l'une quelconque des revendications 1 à 3, dans lequel le composé de céfixime est le céfixime.

5. Procédé selon l'une quelconque des revendications 1, 3 et 4, dans lequel l'antibactérien autre que les composés de céfixime est choisi parmi les groupes d'antibiotiques suivants : les sulfamides, les tétracyclines, les aminoglycosides, les macrolides, les cétolides, les quinolones, les lincomycines et les glycopeptides.
